# EUROPEAN PATENT APPLICATION

(11) **EP 3 508 208 A1**
(43) Date of publication of application: **10.07.2019**
(21) Application number: 17846799.9
(22) Date of filing: 09.06.2017
(51) Int. Cl.: A61K 35/744, A61K 35/747, A23L 33/135, A61K 9/00, C12N 1/20, A61K 35/74, C12R 1/01

(54) **COMPOSITION, FOR PREVENTING HAIR LOSS OR PROMOTING HAIR GROWTH, COMPRISING STRAINS SHOWING LIPOLYSIS EFFECT**

(30) Priority: 30.08.2016 KR 20160111042; 19.01.2017 KR 20170009282
(71) Applicant: Coenbio Co., Ltd., Seongnam-si, Gyeonggi-do 13207 (KR)
(72) Inventor: YUM, Kyu Jin, Anyang-si Gyeonggi-do 14106 (KR)
(74) Representative: Cabinet Beau de Loménie
(86) International application number: PCT/KR2017/005989
(87) International publication number: WO 2018/043874

(57) **Abstract**

The present invention relates to a novel Leuconostoc holzapfelii Ceb-kc-003 (deposited as Korea Accession Number accession KCCM11830P) strain and a composition comprising the same. The novel strain has abilities of preventing hair loss, promoting hair growth, or improving sexual function.

## Description

### [Technical Field]

The present invention relates to a composition for preventing hair loss or promoting hair growth including a strain having lipolysis ability.

### [Background Art]

With the development of modern society, the number of alopecia patients has increased rapidly. Particularly, the age of alopecia patients has dropped gradually. 60% of people who require hair care service are in their twenties and thirties. The number of patients suffering from alopecia is estimated to reach almost 10 million, causing serious socioeconomic problems.

Reports say that people with severe hair loss are more likely to experience psychological depression and anxiety, perceive high levels of stress, are faced with difficulty in their relationships with other people and the opposite sex, and fail to maintain good family relationships. Thus, hair loss has become a matter of concern in health science.

Although the exact cause of hair loss is clearly unknown, reports say that hair loss is possibly caused by genetic, hormonological and immunological abnormalities.

Minoxidil for transdermal application and Finasteride for oral administration approved by the U.S. Food and Drug Administration (FDA) are being currently widely used as therapeutic agents for alopecia. However, these therapeutic agents are limited in their use because of their attendant side effects such as cardiovascular disorders, skin irritation, decreased sexual function, and severe teratogenesis.

Previous studies reported that galenic preparations can ameliorate alopecia, but the results are still unsatisfactory. In recent years, some studies and patents reported that the use of fermentation products of plant extracts and hot water-extracted herbal materials such as fleeceflower root by lactic acid bacteria belonging to the genus *Lactobacillus* can promote hair growth. However, most of the results were obtained from experimental rats and no research has been conducted on people who had suffered or were suffering hair loss.

### [Disclosure]

### [Technical Problem]

The present invention is aimed at providing a composition that is effective in preventing hair loss or promoting hair growth or a novel strain that is isolated from kimchi and is effective in decomposing fat.

The present invention is aimed at providing a composition for preventing hair loss or promoting hair growth that can be used safely without causing side effects.

### [Technical Solution]

One embodiment of the present invention provides a composition for preventing hair loss or promoting hair growth including a strain having lipolysis ability.

The strain having lipolysis ability may be a lactic acid bacterial strain isolated from kimchi. Specifically, the strain may be one belonging to the genus *Leuconostoc* and *Lactobacillus.* More specifically, the strain may be *Leuconostoc holzapfelii*, *Leuconostoc mesenteroides* or *Lactobacillus sakei.* Even more specifically, the strain may be *Leuconostoc holzapfelii* Ceb-kc-003 (KCCM11830P), *Leuconostoc mesenteroides* (KCCM11827P) or *Lactobacillus sakei* (KCCM11841P).

*Leuconostoc holzapfelii* Ceb-kc-003 was deposited with the Korean Culture Center of Microorganisms (120-861, Hongje-2ga-Gil, Seodaemun-Ku, Seoul, Korea) on April 11, 2016 and assigned accession number KCCM11830P. The strain is effective in preventing hair loss, promoting hair growth, improving sexual function, lowering cholesterol levels, and/or decomposing fat.

*Leuconostoc mesenteroides* (KCCM11827P) was deposited with the Korean Culture Center of Microorganisms (120-861, Hongje-2ga-Gil, Seodaemun-Ku, Seoul, Korea) on April 6, 2016 and assigned accession number KCCM11827P. The strain is effective in preventing hair loss, promoting hair growth, improving sexual function, lowering cholesterol levels, and/or decomposing fat.

*Lactobacillus sakei* (KCCM11841P) was deposited with the Korean Culture Center of Microorganisms (120-861, Hongje-2ga-Gil, Seodaemun-Ku, Seoul, Korea) on June 1, 2016 and assigned accession number KCCM11841P. The strain is effective in preventing hair loss, promoting hair growth, improving sexual function, lowering cholesterol levels, and/or decomposing fat.

The strain having lipolysis ability may be used *per* se or in the form of a culture, concentrate or dried product thereof. Accordingly, the composition may include the strain having lipolysis ability or a culture, concentrate or dried product thereof.

The strain having lipolysis ability can be cultured by any suitable method known in the art. A natural or synthetic medium can be used to culture the strain. Examples of carbon sources of the medium include, but are not limited to, glucose, sucrose, dextrin, glycerol, and starch. Examples of nitrogen sources of the medium include, but are not limited to, peptone, meat extract, yeast extract, dried yeast, soybean, ammonium salt, nitrate, and other organic and inorganic nitrogenous compounds. One or more inorganic salts may be added to the medium. Examples of such inorganic salts include, but are not limited to, magnesium, manganese, calcium, iron, and potassium salts. The medium may further include one or more compounds selected from amino acids, vitamins, nucleic acids, and compounds related thereto. The strain may be cultured at a temperature of 20 to 40°C for 12 hours to 4 days. As an example, one or more galenic preparations, an extract thereof or a mixture thereof may be added to and cultured in the culture medium. Examples of the galenic preparations include green kernel black bean, *Cynanchum wilfordii* root, cooked Rehmannia, licorice root, Szechuan lovage root, Eucommia bark, Chinese cinnamon bark, Chinese angelica root, sweetflag rhizome, fleeceflower root, leafy twig of arorvitae, ginger, arbor-vitae seed, fruit of Szechuan pepper, bugbane rhizome, and vitex fruit. The use of the culture further improves the effect of the strain to prevent hair loss or promote hair growth. The addition of green kernel black bean and/or *Cynanchum wilfordii* root to the culture medium is particularly preferred. The green kernel black bean and/or *Cynanchum wilfordii* root may be added in an amount of 0.01% by weight (wt%) to 10 wt%, specifically 0.01 wt% to 5 wt%.

The culture of the strain having lipolysis ability may be a culture broth containing the bacterial cells. The culture may also be obtained by removal of the supernatant from the culture broth or concentration of the culture broth. The composition of the culture may further include not only one or more typical ingredients necessary for the culture of strains having lipolysis ability but also one or more ingredients exerting a synergistic effect on the growth of the strains. The composition of the culture can be readily determined by those skilled in the art.

The strain may be in a liquid or dry state. The strain can be dried by any suitable technique known in the art. Examples of such drying techniques include, but are not limited to, air drying, natural drying, spray drying, and freeze drying.

The composition is effective in preventing hair loss or promoting hair growth. The composition is also effective in improving sexual function or decomposing fat. The composition may be used as a pharmaceutical composition. Alternatively, the composition may be used as a food, health food, health supplement food or health functional food composition. One embodiment of the pharmaceutical composition may be a quasi-drug or a pharmaceutical preparation.

As an example, the composition may further include one or more galenic ingredients that are effective in preventing hair loss or promoting hair growth or one or more pharmaceutical ingredients. Examples of the galenic ingredients include galenic preparations such as green kernel black bean, *Cynanchum wilfordii* root, cooked Rehmannia, licorice root, Szechuan lovage root, Eucommia bark, Chinese cinnamon bark, Chinese angelica root, sweetflag rhizome, red fleeceflower root, leafy twig of arorvitae, Acanthopanax senticosus, pine leaf and flower, buckwheat, Anemarrhena rhizome, ginger, arbor-vitae seed, fruit of Szechuan pepper, bugbane rhizome, and vitex fruit, extracts thereof, and mixtures thereof. Examples of the pharmaceutical ingredients include Minoxidil or Finasteride.

A further embodiment of the present invention relates to a composition for preventing hair loss or promoting hair growth including a strain having lipolysis ability and a strain capable of lowering cholesterol levels. The strain capable of lowering cholesterol levels may be derived from cheonggukjang. Specifically, the strain capable of lowering cholesterol levels may be, for example, one belonging to the genus *Brevibacillus*, *Lactobacillus, Lactococcus, Propionibacterium, Enterocuccus* or *Bifidobacterium.* More specifically, the strain capable of lowering cholesterol levels may be, for example, *Lactobacillus fermentum, Brevibacillus reuszeri* or *Enterocuccus faecium.* In one embodiment, *Brevibacillus reuszeri* Ceb-ch-003 (KCCM11911P), *Enterocuccus faecium* Ceb-ch-001 (KCCM11909P) and/or *Lactobacillus fermentum* Ceb-ch-002 (KCCM11910P) may be used in combination with the strain having lipolysis ability described herein.

*Brevibacillus reuszeri* Ceb-ch-003 was isolated from cheonggukjang and was deposited with the Korean Culture Center of Microorganisms (120-861, Hongje-2ga-Gil, Seodaemun-Ku, Seoul, Korea) on October 7, 2016 and assigned accession number KCCM11911P. The strain is effective in improving sexual function or lowering cholesterol levels.

*Enterocuccus faecium* Ceb-ch-001 was isolated from cheonggukjang and was deposited with the Korean Culture Center of Microorganisms (120-861, Hongje-2ga-Gil, Seodaemun-Ku, Seoul, Korea) on October 7, 2016 and assigned accession number KCCM11909P. The strain is effective in improving sexual function or lowering cholesterol levels.

*Lactobacillus fermentum* Ceb-ch-002 was isolated from cheonggukjang was deposited with the Korean Culture Center of Microorganisms (120-861, Hongje-2ga-Gil, Seodaemun-Ku, Seoul, Korea) on October 7, 2016 and assigned accession number KCCM11910P. The strain is effective in improving sexual function or lowering cholesterol levels.

The composition further includes the strain capable of lowering cholesterol levels at a concentration of 2.5 × 10⁴ to 2.5 × 10¹⁰ CFU/ml, preferably 5 × 10⁵ to 5 × 10⁸ CFU/ml. The strain capable of lowering cholesterol levels may be present in an amount of 0.05 wt% to 50 wt%, based on the weight of the composition.

The composition may further include collagen.

The composition includes the strain having lipolysis ability at a concentration of 5 × 10⁴ to 5 × 10¹⁰ CFU/ml, preferably 1 × 10⁶ to 1 × 10⁹ CFU/ml.

The composition of the present invention may further include a pharmaceutically or sitologically acceptable carrier. In this case, the composition may be formulated with the carrier to provide a food or pharmaceutical drug.

As used herein, the term "pharmaceutically or sitologically acceptable carrier" refers to a carrier or diluent that causes no irritation to target organisms and does not deteriorate the biological activity and properties of the strain.

The composition may be formulated into various preparations for oral or parenteral administration. Preferably, the composition is formulated into a liquid oral solution.

When formulated into a liquid solution, the pharmaceutically or sitologically acceptable carrier may be selected from saline solution, sterilized water, buffered saline, albumin injection solution, dextrose solution, maltodextrin solution, glycerol, and mixtures thereof that are sterile and biocompatible. If necessary, one or more general additives, such as antioxidants, buffer solutions, and bacteriostatic agents may be added to the composition. The composition of the present invention may be formulated into liquid preparations (such as aqueous solutions, suspensions or emulsions), pills, capsules, granules, and tablets. In this case, the composition of the present invention may further include one or more additives selected from diluents, dispersants, surfactants, binders, and lubricants. A binding agent, an emulsifier or preservative may be further added to the composition to prevent the quality of the composition from deteriorating. The composition of the present invention may further include one or more additives selected from amino acids, vitamins, enzymes, flavoring agents, non-protein nitrogen compounds, silicates, buffers, extractants, and oligosaccharides. The strain having lipolysis ability may be administered in the form of a liquid. In this case, the strain having lipolysis ability at a concentration of 5 × 10⁴ to 5 × 10⁸ CFU/ml, preferably 1 × 10⁶ to 1 × 10⁸ CFU/ml, may be administered 1 to 4 times (30 ml to 100 ml each time) daily. As an example, the strain having lipolysis ability at a concentration of 1 × 10⁶ to 1 × 10⁸ CFU/ml may be administered 2 to 4 times (50 ml each time) daily. More specifically, the strain having lipolysis ability at a concentration of 1 × 10⁶ to 1 × 10⁸ CFU/ml may be administered once or twice (50 ml to 100 ml each time) daily. Even more specifically, the strain having lipolysis ability at a concentration of 1 × 10⁶ to 1 × 10⁸ CFU/ml may be administered in an amount of 50 ml to 100 ml once 30 minutes before breakfast and in an amount of 50 ml to 100 ml once 30 minutes before dinner or before bed. The strain capable of lowering cholesterol levels at a concentration of 2.5 × 10⁴ to 2.5 × 10⁸ CFU/ml, preferably 5 × 10⁵ to 5 × 10⁷ CFU/ml, may be administered 1 to 4 times (15 ml to 50 ml each time) daily. As an example, the strain capable of lowering cholesterol levels at a concentration of 5 × 10⁵ to 5 × 10⁷ CFU/ml may be administered 2 to 4 times (25 ml to 50 ml each time) daily. More specifically, the strain capable of lowering cholesterol levels at a concentration of 5 × 10⁵ to 5 × 10⁷ CFU/ml may be administered once or twice (25 ml to 100 ml each time) daily. Even more specifically, the strain capable of lowering cholesterol levels at a concentration of 5 × 10⁵ to 5 × 10⁷ CFU/ml may be administered in an amount of 20 ml to 100 ml once 30 minutes before breakfast and in an amount of 20 ml to 100 ml once 30 minutes before dinner or before bed. The strain capable of lowering cholesterol levels may be administered simultaneously with the strain having lipolysis ability. Alternatively, the two strains may be administered sequentially. The administration cycle and dose of the strain capable of lowering cholesterol levels may be different from those of the strain having lipolysis ability.

Examples of oral preparations including the strain having lipolysis ability include tablets, troches, lozenges, water-soluble or oily suspensions, powders, granules, emulsions, hard or soft capsules, syrups, and elixirs. The composition of the present invention may be formulated into desired preparations such as tablets and capsules. In this case, the composition may include: a binder such as lactose, saccharose, sorbitol, mannitol, starch, amylopectin, cellulose or gelatin; an excipient such as dicalcium phosphate; a disintegrant such as corn starch or sweet potato starch; or a lubricant such as magnesium stearate, calcium stearate, sodium stearyl fumarate or polyethylene glycol wax. For capsule preparations, the composition may further include a liquid carrier such as a fatty oil.

The daily dose of the composition, the strain having lipolysis ability or the strain capable of lowering cholesterol levels may vary depending on the weight, age, sex, health condition, main symptoms to be treated, prevented or ameliorated, time and mode of administration, and severity of disease and may be in the range of about 0.0001 mg/kg to about 10 g/kg, specifically about 0.01 mg/kg to about 500 mg/kg. The composition, the strain having lipolysis ability or the strain capable of lowering cholesterol levels may be administered or applied 1 to 6 times, for example, 1 to 4 times, a day.

The strain having lipolysis ability may be present in a liquid or dry state, preferably a liquid state, in the composition. The strain can be dried by any suitable technique known in the art. Examples of such drying techniques include, but are not limited to, air drying, natural drying, spray drying, and freeze drying. The strain having lipolysis ability may be used in the form of a powder. In this case, the strain may be added in an amount of 0.05 to 80 wt%, for example, 0.1 to 70 wt%, 0.1 to 50 wt% or 1 to 30 wt%, based on the weight of the composition.

A further embodiment of the present invention relates to a method for preventing hair loss or promoting hair growth including administering a composition including the strain having lipolysis ability to a subject. The composition including the strain having lipolysis ability is the same as that described in the previous embodiment and a detailed description thereof is thus omitted to avoid duplication.

Another embodiment of the present invention relates to a *Leuconostoc mesenteroides* strain (KCCM11827P) or a *Lactobacillus sakei* strain (KCCM11841P), a culture thereof, a concentrate thereof or a dried product thereof. The strains are lactic acid bacterial strains isolated from kimchi and have the ability to decompose fat. The strains are effective in preventing hair loss, promoting hair growth or decomposing fat.

Another embodiment of the present invention relates to a method for preparing a composition for preventing hair loss or promoting hair growth, including preparing a strain having lipolysis ability, a culture thereof, a concentrate thereof or a dried product thereof and applying a pharmaceutically or sitologically acceptable carrier to the strain or the culture, concentrate or dried product thereof. The strain having lipolysis ability and the culture, concentrate or dried product thereof are the same as those described in the previous embodiment and a detailed description thereof is thus omitted to avoid duplication.

One embodiment of the present invention relates to a composition for ameliorating sexual dysfunction including a strain having lipolysis ability, a culture thereof, a concentrate thereof or a dried product thereof. The sexual dysfunction may be erectile dysfunction. A further embodiment of the present invention relates to a method for preventing or treating sexual dysfunction including administering to a subject in need of such prevention or treatment a therapeutically or prophylactically effective amount of the strain having lipolysis ability or the culture, concentrate or dried product thereof. The therapeutically or prophylactically effective amount is the same as that described above.

### [Advantageous Effects]

The strain having lipolysis ability and the composition including the strain are effective in preventing hair loss, promoting hair growth or improving sexual function.

In addition, the strain having lipolysis ability and the composition including the strain can be used safely without causing side effects to prevent hair loss, promote hair growth, lower cholesterol levels or improve sexual function.

### [Description of Drawings]

FIG. 1 is a histogram comparing the effects of *Leuconostoc holzapfelii* Ceb-kc-003 (KCCM11830P) and another lactic acid bacterial strain on fat decomposition.
FIG. 2 is a histogram comparing the effects of *Leuconostoc holzapfelii* Ceb-kc-003 (KCCM11830P) and another lactic acid bacterial strain on NO production.
FIG. 3 is a histogram comparing the effects of *Lactobacillus sakei* (KCCM11841P) and another lactic acid bacterial strain on fat decomposition.
FIG. 4 is a histogram comparing the effects of *Leuconostoc mesenteroides* KCCM11827P and another lactic acid bacterial strain on fat decomposition.
FIG. 5 shows photographs comparing scalp hair before and ∼3.5 months after taking *Leuconostoc holzapfelii* Ceb-kc-003 (KCCM11830P).
FIG. 6 shows photographs comparing scalp hair before and ∼2.5 and ∼3.5 months after taking *Leuconostoc holzapfelii* Ceb-kc-003 (KCCM11830P).
FIG. 7 shows photographs comparing scalp hair before and ∼1.7 months after taking *Leuconostoc holzapfelii* Ceb-kc-003 (KCCM11830P).
FIG. 8 shows photographs comparing scalp hair before and ∼3 months after taking *Leuconostoc holzapfelii* Ceb-kc-003 (KCCM11830P).
FIG. 9 shows photographs showing changes in the gloss and thickness of hairs and the growth of new bangs after taking *Leuconostoc mesenteroides* (KCCM11827P) for ∼3 months.
FIG. 10 shows photographs comparing scalp hair before and ∼45 days after taking *Leuconostoc mesenteroides* (KCCM11827P).
FIG. 11 shows photographs comparing hair thicknesses, strengths, and colors before and ∼1 month after taking *Leuconostoc mesenteroides* (KCCM11827P).
FIG. 12 shows photographs comparing scalp hair before and ∼27 days after taking *Lactobacillus sakei* (KCCM11841P).
FIG. 13 shows photographs comparing scalp hair before and ∼2 months after taking *Lactobacillus sakei* (KCCM11841P).
FIG. 14 shows photographs comparing scalp hair before and -1.5 months after taking *Lactobacillus sakei* (KCCM11841P).
FIG. 15 shows photographs comparing scalp hair before and ∼2 months after taking a combination of *Leuconostoc mesenteroides* (KCCM11827P) and *Lactobacillus fermentum* Ceb-ch-002 (KCCM11910P).
FIG. 16 shows photographs comparing scalp hair before and ∼1.5 months after taking a combination of *Leuconostoc mesenteroides* (KCCM11827P) and *Lactobacillus fermentum* Ceb-ch-002 (KCCM11910P).
FIG. 17 shows photographs comparing scalp hair before and ∼1.5 months after taking a combination of *Lactobacillus sakei* (KCCM11841P) and *Lactobacillus fermentum* Ceb-ch-002 (KCCM11910P).
FIG. 18 shows photographs comparing scalp hair before and ∼3 months after taking a combination of *Lactobacillus sakei* (KCCM11841P) and *Lactobacillus fermentum* Ceb-ch-002 (KCCM11910P).
FIG. 19 is a histogram comparing the ability of *Lactobacillus fermentum* (KCCM11910P) isolated from cheonggukjang, Korean fermented soybean, to remove cholesterol with that of another lactic acid bacterial strain.

### [Mode for Invention]

The present invention will be described in more detail with reference to the following examples. However, these examples are provided for illustrative purposes only and the present invention is not limited thereto.

### Example 1-3: Isolation of Leuconostoc holzapfelii strain Ceb-kc-003 (KCCM11830P), Leuconostoc mesenteroides strain (KCCM11827P) and Lactobacillus sakei strain (KCCM11841P) from kimchi and identification of the strains

### (1) Sampling and strain isolation

A sample was taken from kimchi, a traditional Korean fermented food, diluted stepwise, plated on a BHI solid medium (Difco, USA) supplemented with 3% sodium chloride, and cultured at 37°C for 24 h. A dominant strain was isolated from the sample. Colonies were selected and passaged three times in fresh media. The pure cultured bacterial strain was placed in a medium supplemented with 20% glycerol and stored at ≤ -70°C.

### (2) Investigation of morphological and taxological properties

The isolated strain was identified. To this end, the strain was primarily morphologically and biochemically investigated. The strain was found to be morphologically Gram-positive by Gram staining. The taxological properties of the strain were analyzed by 16s rRNA partial sequencing. As a result, the isolated strain was found to have a homology of 99% with *Leuconostoc holzapfelii.*

The newly isolated strain *Leuconostoc holzapfelii* Ceb-kc-003 was deposited with the Korean Culture Center of Microorganisms (120-861, Hongje-2ga-Gil, Seodaemun-Ku, Seoul, Korea) on April 11, 2016 and assigned accession number KCCM11830P.

A dominant strain was isolated from another kimchi sample after culture under the conditions described above. As a result of morphological and biochemical investigation, the strain was found to have a homology of 99% with *Leuconostoc mesenteroides.*

The newly isolated strain *Leuconostoc mesenteroides* was deposited with the Korean Culture Center of Microorganisms (120-861, Hongje-2ga-Gil, Seodaemun-Ku, Seoul, Korea) on April 6, 2016 and assigned accession number KCCM11827P.

A dominant strain was isolated from another kimchi sample after culture under the conditions described above. As a result of morphological and biochemical investigation, the strain was found to have a homology of 99% with *Lactobacillus sakei.*

The newly isolated strain *Lactobacillus sakei* was deposited with the Korean Culture Center of Microorganisms (120-861, Hongje-2ga-Gil, Seodaemun-Ku, Seoul, Korea) on June 1, 2016 and assigned accession number KCCM11841P.

The strains were freeze-dried and powdered.

### (3) Preparation of culture solutions of the strains

A culture solution of *Leuconostoc holzapfelii* Ceb-kc-003 was prepared by the following procedure (Example 1):

2 kg of dextrose, 1.5 kg of whole milk powder, and 0.05 kg of an yeast extract were added to 100 liters of purified water. The mixture was sterilized in an autoclave at 121°C for 15-30 min and cooled to around 35 °C. Thereafter, the sterile mixture was inoculated with 0.2-0.4 liters of *Leuconostoc holzapfelii* Ceb-kc-003, followed by culture at around 35°C for 2-3 days.

Culture solutions of *Leuconostoc mesenteroides* (KCCM11827P) and *Lactobacillus sakei* (KCCM11841P) were prepared in the same manner as described above for the culture solution of *Leuconostoc holzapfelii* Ceb-kc-003 (Examples 2-3, respectively).

### Example 4: Effect of the Leuconostoc holzapfelii strain Ceb-kc-003 (KCCM11830P) on fat decomposition

In this example, the effect of the *Leuconostoc holzapfelii* strain Ceb-kc-003 (KCCM11830P) on fat decomposition was examined. To this end, 1000 cc of pork fat was treated with 20-100 ml of culture solutions of the strain, followed by shaking at 37°C for 24 h. This procedure was repeated for *Lactobacillus plantarum* as a control.

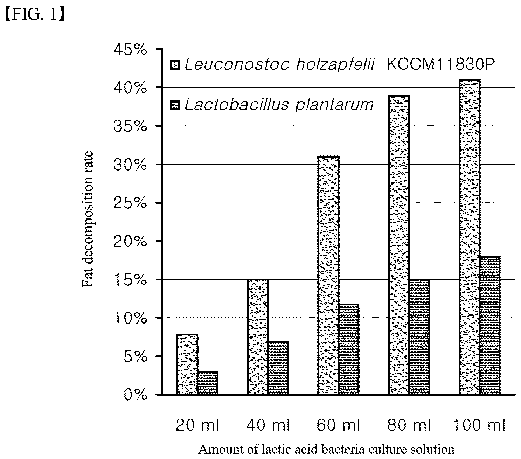

The results are shown in FIG. 1.

From these results, it can be concluded that the powder of the *Leuconostoc holzapfelii* strain Ceb-kc-003 (KCCM11830P) is significantly effective in fat decomposition in proportion to the strain concentration compared to the other lactic acid bacterial species *Lactobacillus plantarum.*

### Example 5: Effects of the Leuconostoc holzapfelii strain Ceb-kc-003 (KCCM11830P) on improving sexual function and preventing sexual dysfunction

The powders of the *Leuconostoc holzapfelii* strain Ceb-kc-003 (KCCM11830P) at concentrations of 1 × 10⁶ to 1 × 10⁸ CFU/ml were administered orally to a total of 10 male smokers or non-smokers with or without sexual dysfunction, aged 45-70 years, once or twice (50 ml to 100 ml each time) daily over a total of 3 months. The frequency of erections in the early morning, erectility, and duration of erection were self-evaluated before and at 1-month intervals after administration of the strain.

4 out of the 10 men answered questionnaires for 1-3 months after taking. The results are shown in Table 1.

**[Table 1]**

| Data on effects of the strain on improving in sexual function | | | | |
|---|---|---|---|---|
| **Subject Time** | **Male non-smoker at age 45** | **Male non-smoker at age 54** | **Male smoker at age 63** | **Male smoker at age 57** |
| Before taking | Good erectility | Good erectility | Impotence | Poor erectility |
| 1 month after taking | Increased frequency of erections in the early morning | Good erectility | Impotence | Poor erectility |
| 2 months after taking | Increased frequency of erections in the early morning | Increased duration of erection | Erection in the early morning | Increased frequency of erections in the early morning |
| 3 months after taking | Increased duration of erection | Increased frequency of erections | Increased frequency of erections in the early morning | Increased duration of erection |

The above results indicate that continuous taking of the powder of the *Leuconostoc holzapfelii* strain Ceb-kc-003 is effective in preventing and treating sexual dysfunction.

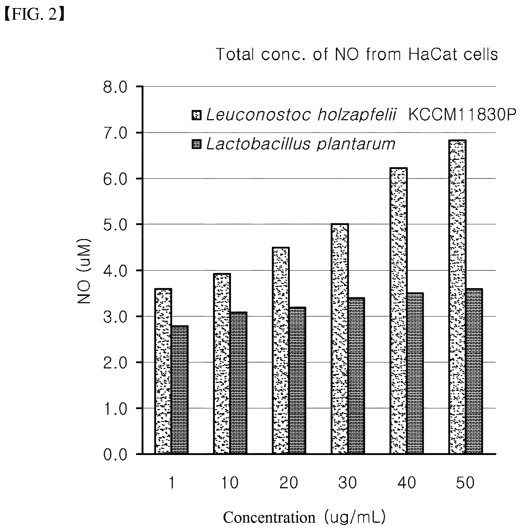

In addition, the concentrations of NO released from Hacat cells for 24 h were evaluated. NO is helpful in improving erectility due to its ability to increase blood circulation and dilate capillaries. The concentrations of NO produced after taking the novel strain of the present invention were compared with those after taking *Lactobacillus plantarum.* This experiment was conducted in triplicate and the data were averaged. The results are shown in FIG. 2.

### Example 6: Effect of the Lactobacillus sakei strain (KCCM11841P) on fat decomposition

In this example, the effect of the *Lactobacillus sakei* strain (KCCM11841P) on fat decomposition was examined. To this end, 1000 cc of pork fat was treated with 20-100 ml of culture solutions of the strain, followed by shaking at 37°C for 24 h. This procedure was repeated for *Lactobacillus plantarum* as a control.

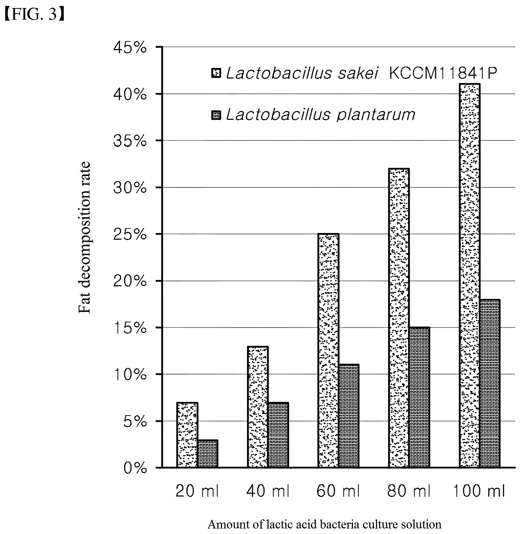

The results are shown in Table 2 and FIG. 3.

**[Table 2]**

| | *Lactobacillus sakei* (KCCM11841P) | *Lactobacillus plantarum* |
|---|---|---|
| 20 ml | 7% | 3% |
| 40 ml | 13% | 7% |
| 60 ml | 25% | 11% |
| 80 ml | 32% | 15% |
| 100 ml | 41% | 18% |

From these results, it can be concluded that the powder of the *Lactobacillus sakei* strain (KCCM11841P) is significantly effective in fat decomposition in proportion to the strain concentration compared to the other lactic acid bacterial species *Lactobacillus plantarum.*

### Example 7: Effect of the Leuconostoc mesenteroides strain (KCCM11827P) on fat decomposition

In this example, the effect of the *Leuconostoc mesenteroides* strain (KCCM11827P) on fat decomposition was examined. To this end, 1000 cc of pork fat was treated with 20-100 ml of culture solutions of the strain, followed by shaking at 37°C for 24 h. This procedure was repeated for *Lactobacillus plantarum* as a control.

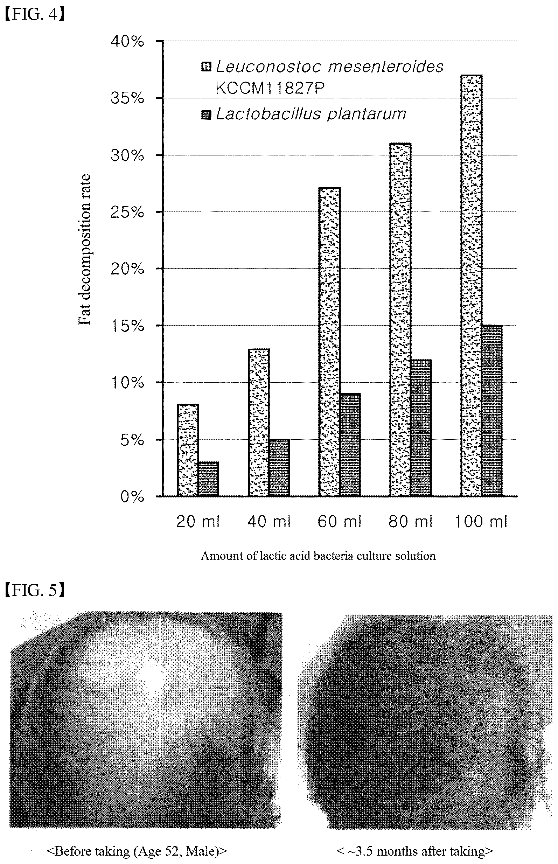

The results are shown in Table 3 and FIG. 4.

**[Table 3]**

| | *Leuconostoc mesenteroides* (KCCM11827P) | *Lactobacillus plantarum* |
|---|---|---|
| 20 ml | 8% | 3% |
| 40 ml | 13% | 5% |
| 60 ml | 27% | 9% |
| 80 ml | 31% | 11% |
| 100 ml | 37% | 15% |

From these results, it can be concluded that the powder of the *Leuconostoc mesenteroides* strain (KCCM11827P) is significantly effective in fat decomposition in proportion to the strain concentration compared to the other lactic acid bacterial species *Lactobacillus plantarum.*

### Example 8: Preventive effect of the Leuconostoc holzapfelii strain Ceb-kc-003 (KCCM11830P) on hair loss

The culture solution of the *Leuconostoc holzapfelii* strain Ceb-kc-003 (KCCM11830P) prepared in Example 1 at a concentration of 1 × 10⁶ to 1 × 10⁸ CFU/ml was administered orally to a total of 5 men aged 40-85 years and one woman who suffered from alopecia twice daily (50-100 ml each time) over 1-4 months.

The numbers of hairs lost were counted before and 10, 20, and 30 days after taking the strain. The patients were instructed to wash their hair before hair counting.

The experimental results are shown in Table 4.

**[Table 4]**

| Numbers of hairs lost before and after taking | | | | |
|---|---|---|---|---|
| **Subject Time** | **Man at age 45** | **Man at age 52** | **Woman at age 38** | **Men at age 57** |
| Before taking | 60 hairs lost | 43 hairs lost | 74 hairs lost | 23 hairs lost |
| 10 days after taking | 40 hairs lost | 32 hairs lost | 55 hairs lost | 10 hairs lost |
| 20 days after taking | 22 hairs lost | 15 hairs lost | 23 hairs lost | 4 hairs lost |
| 30 days after taking | 12 hairs lost | 5 hairs lost | 8 hairs lost | 2 hairs lost |

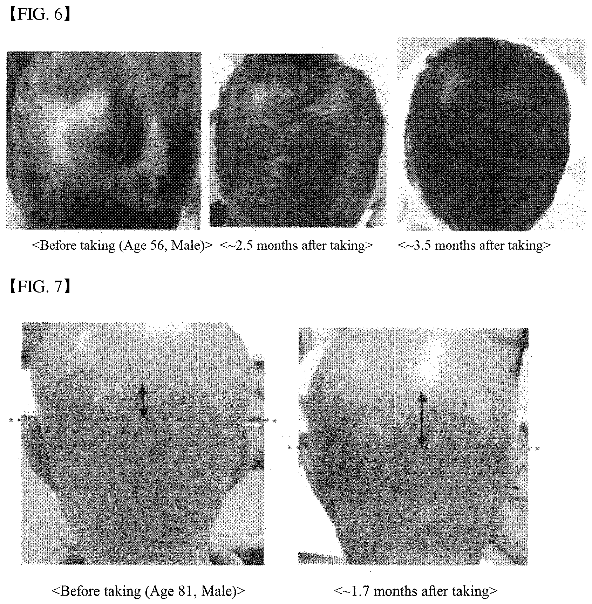

The hairs of the male patients before and at the predetermined time points after administration of the strain were photographed to measure their thicknesses, numbers, and glosses. Some of the results are shown in FIGs. 5-8.

These results indicate that the *Leuconostoc holzapfelii* strain Ceb-kc-003 (KCCM11830P) is effective in preventing hair loss and markedly improving the thickness, number, and gloss of hairs. Therefore, it can be concluded that the *Leuconostoc holzapfelii* strain Ceb-kc-003 (KCCM11830P) is effective in treating hair loss.

### Example 9: Preventive effect of the Leuconostoc mesenteroides strain (KCCM11827P) on hair loss

The culture solution of the *Leuconostoc mesenteroides* strain (KCCM11827P) prepared in Example 2 at a concentration of 1 × 10⁶ to 1 × 10⁸ CFU/ml was administered orally to a woman aged 43 years, a man aged 60 years and a woman aged 49 years who suffered from alopecia twice daily (50-100 ml each time) over 1-3 months.

The numbers of hairs lost were counted before and 10, 20, and 30 days after taking the strain. The patients were instructed to wash their hair before hair counting.

The experimental results are shown in Table 5.

**[Table 5]**

| Numbers of hairs lost before and after taking | | | |
|---|---|---|---|
| **Subject Time** | **Woman at age 43** | **Man at age 60** | **Woman at age 49** |
| Before taking | 39 hairs lost | 43 hairs lost | 25 hairs lost |
| 10 days after taking | 35 hairs lost | 32 hairs lost | 21 hairs lost |
| 20 days after taking | 29 hairs lost | 28 hairs lost | 20 hairs lost |
| 30 days after taking | 21 hairs lost | 16 hairs lost | 12 hairs lost |

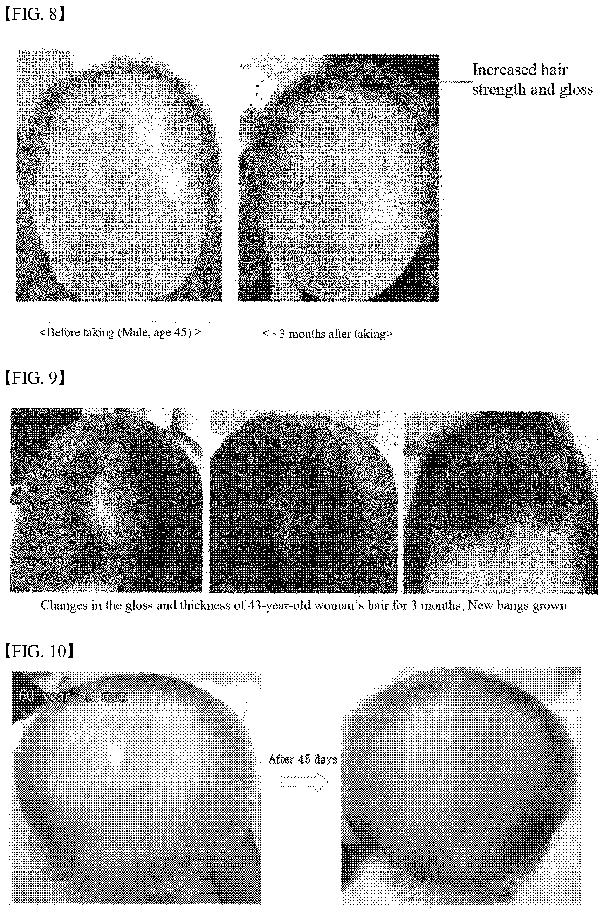

The hairs of the patients before and at the predetermined time points after administration of the strain were photographed to measure their thicknesses, numbers, and glosses. Some of the results are shown in FIGs. 9-11.

These results indicate that the *Leuconostoc mesenteroides* strain (KCCM11827P) is effective in preventing hair loss and markedly improving the thickness, number, and gloss of hairs. Therefore, it can be concluded that the *Leuconostoc holzapfelii* strain Ceb-kc-003 (KCCM11830P) is effective in treating hair loss.

### Example 10: Preventive effect of the Lactobacillus sakei strain (KCCM11841P) on hair loss

The culture solution of the *Lactobacillus sakei* strain (KCCM11841P) prepared in Example 3 at a concentration of 1 × 10⁶ to 1 × 10⁸ CFU/ml was administered orally to a man aged 52 years, a woman aged 42 years and a man aged 60 years who suffered from alopecia twice daily (50-100 ml each time) over 0.8-3 months.

The numbers of hairs lost were counted before and 10, 20, and 27 days after taking the strain. The patients were instructed to wash their hair before hair counting.

The experimental results are shown in Table 6.

**[Table 6]**

| Numbers of hairs lost before and after taking | | | |
|---|---|---|---|
| **Subject Time** | **Man at age 52** | **Woman at age 42** | **Man at age 60** |
| Before taking | 47 hairs lost | 40 hairs lost | 47 hairs lost |
| 10 days after taking | 40 hairs lost | 32 hairs lost | 37 hairs lost |
| 20 days after taking | 35 hairs lost | 25 hairs lost | 26 hairs lost |
| 27 days after taking | 30 hairs lost | 24 hairs lost | 16 hairs lost |

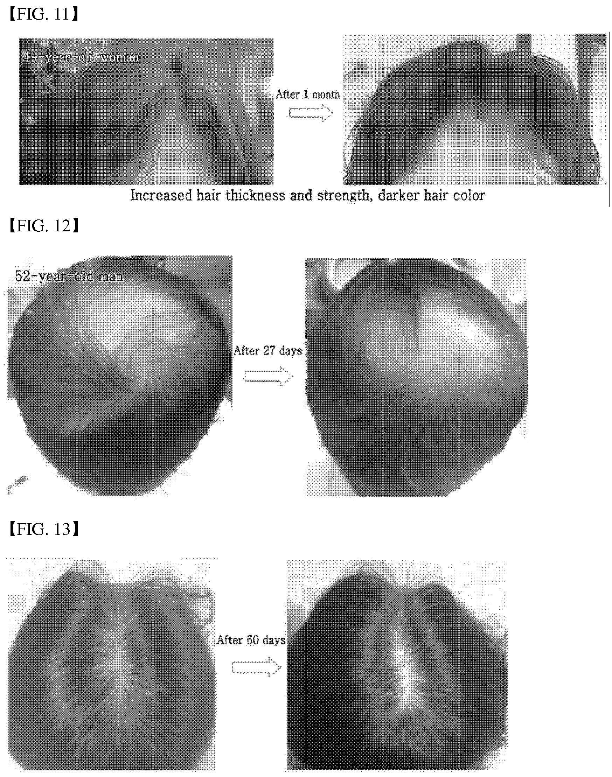

The hairs of the patients before and at the predetermined time points after administration of the strain were photographed to determine whether the patients' scalps were improved and to measure the hair thicknesses, numbers, and glosses. Some of the results are shown in FIGs. 12-14.

These results indicate that the *Lactobacillus sakei* strain (KCCM11841P) is effective in preventing hair loss, improving the condition of scalp, and markedly improving the thickness, number, and gloss of hair. Therefore, it can be concluded that the *Lactobacillus sakei* strain (KCCM11841P) is effective in treating hair loss.

### Example 11: Isolation and identification of Lactobacillus fermentum strain Ceb-ch-002 (KCCM11910P)

### (1) Sampling and strain isolation

A sample was taken from cheonggukjang, a Korean fermented soybean, diluted stepwise, plated on a BHI solid medium (Difco, USA) supplemented with 3% sodium chloride, and cultured at 37°C for 24 h. A dominant strain was isolated from the sample. Colonies were selected and passaged three times in fresh media. The pure cultured bacterial strain was placed in a medium supplemented with 20% glycerol and stored at ≤ -70°C.

### (2) Investigation of morphological and taxological properties

The isolated strain was identified. To this end, the strain was primarily morphologically and biochemically investigated. The strain was found to be morphologically Gram-positive by Gram staining. The taxological properties of the strain were analyzed by 16s rRNA partial sequencing. As a result, the isolated strain was found to have a homology of 99% with *Lactobacillus fermentum.*

The newly isolated strain *Leuconostoc fermentum* Ceb-ch-002 was deposited with the Korean Culture Center of Microorganisms (120-861, Hongje-2ga-Gil, Seodaemun-Ku, Seoul, Korea) on October 7, 2016 and assigned accession number KCCM11910P.

The strain was freeze-dried and powdered.

### (3) Preparation of culture solution of the strain

A culture solution of *Lactobacillus fermentum* (KCCM11910P) was prepared by the following procedure:

2 kg of dextrose, 1.5 kg of whole milk powder, and 0.05 kg of an enzyme extract were added to 100 liters of purified water. The mixture was sterilized in an autoclave at 121°C for 15-30 min and cooled to around 35 °C. Thereafter, the sterile mixture was inoculated with 0.2-0.4 liters of *Lactobacillus fermentum* Ceb-ch-002, followed by culture at around 35°C for 2-3 days.

### Example 12: Effect of the Lactobacillus fermentum strain Ceb-ch-002 (KCCM11910P) on reducing cholesterol levels

The effect of the powder of the *Lactobacillus fermentum* strain Ceb-ch-002 (KCCM11910P) on reducing cholesterol levels was examined. To this end, solutions of the freeze-dried powder of the *Lactobacillus fermentum* strain Ceb-ch-002 (KCCM11910P) (1-5 mg/ml) and solutions of a powder of *Lactobacillus plantarum* (1-5 mg/ml) as controls were used.

### <Ability to remove cholesterol>

The ability of the strain to remove cholesterol was specifically measured by the following procedure.

30 ml of an MRS culture ground was added to a 50 ml Falcon tube, inoculated with the test strain (glycerol stock) at a density of 1 × 10⁷ counts/ml, and cultured at 30°C for 24 h. 300 µl of the culture solution was added to 30 ml of an MRS culture ground containing 350 mg/L cholesterol (Wako Pure Chemical) and 0.2% bile acid (w/v). After culture at 37°C for 24 h, the culture solution was subjected to centrifugal separation at 8,000 rpm and 4°C for 10 min. The cholesterol level of the supernatant was measured using a Determiner FC (Kyowa Medex Co., Ltd.). The level (%) of cholesterol removed from the culture ground was calculated by subtracting the remaining cholesterol level from the initial cholesterol level of the cholesterol culture ground.

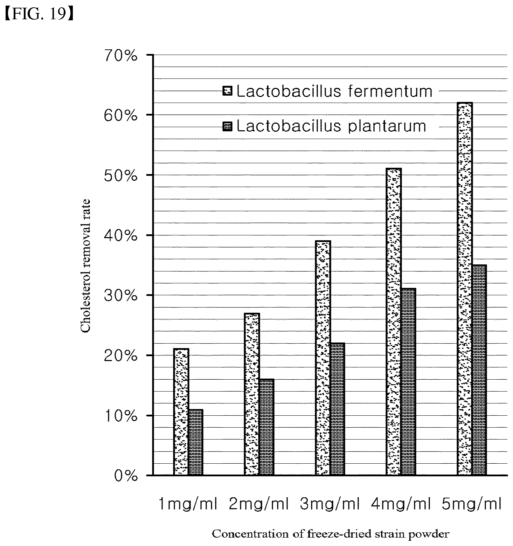

The results are shown in FIG. 19 and Table 7. This experiment was conducted in triplicate and the data were averaged.

**[Table 7]**

| | *Lactobacillus fermentum* Ceb-ch-002 (KCCM11910P) | *Lactobacillus plantarum* |
|---|---|---|
| 1 mg/ml | 21% | 11% |
| 2 mg/ml | 27% | 16% |
| 3 mg/ml | 39% | 22% |
| 4 mg/ml | 51% | 31% |
| 5 mg/ml | 62% | 35% |

### Example 13: Effect of combination of the Leuconostoc mesenteroides strain (KCCM11827P) and the Lactobacillus fermentum strain Ceb-ch-002 (KCCM11910P) on hair loss

The culture solution of the *Leuconostoc mesenteroides* strain (KCCM11827P) prepared in Example 2 at a concentration of 0.5 × 10⁶ to 0.5 × 10⁸ CFU/ml was combined with the culture solution of the *Lactobacillus fermentum* strain (KCCM11910P) prepared in Example 3 at a concentration of 0.5 × 10⁶ to 0.5 × 10⁸ CFU/ml. The combination of the two strain culture solutions was administered orally to a man aged 56 and a man aged 55 who suffered from alopecia twice (50-100 ml each time) daily over 1-3 months.

The numbers of hairs lost were counted before and 10, 20, and 40 days after taking the strains. The patients were instructed to wash their hair before hair counting.

The experimental results are shown in Table 8.

**[Table 8]**

| Numbers of hairs lost before and after taking | | |
|---|---|---|
| **Subject Time** | **Man at age 56** | **Man at age 55** |
| Before taking | 40 hairs lost | 50 hairs lost |
| 10 days after taking | 34 hairs lost | 42 hairs lost |
| 20 days after taking | 26 hairs lost | 28 hairs lost |
| 40 days after taking | 14 hairs lost | 11 hairs lost |

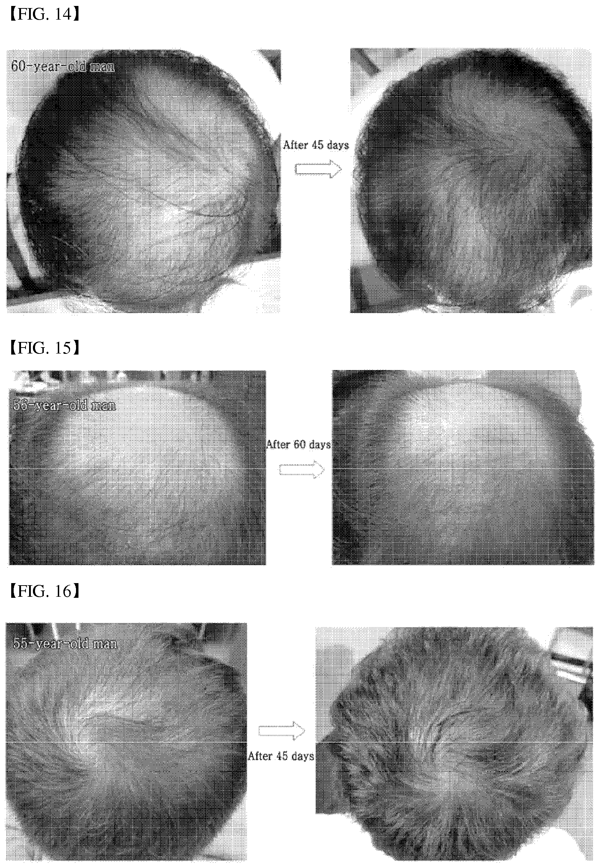

The hairs of the patients before and at the predetermined time points after administration of the strains were photographed to measure their thicknesses, numbers, and glosses. Some of the results are shown in FIGs. 15-16.

These results indicate that the combination of the *Leuconostoc mesenteroides* strain (KCCM11827P) and the *Lactobacillus fermentum* strain (KCCM11910P) is particularly effective in preventing hair loss and markedly improving the thickness, number, and gloss of hairs. Therefore, it can be concluded that the combined administration of the two strains is effective in treating hair loss compared to single administration of the *Leuconostoc mesenteroides* strain (KCCM11827P).

### Example 14: Preventive effect of combination of the Lactobacillus sakei strain (KCCM11841P) and the Lactobacillus fermentum strain Ceb-ch-002 (KCCM11910P) on hair loss

The culture solution of the *Lactobacillus sakei* strain (KCCM11841P) prepared in Example 3 at a concentration of 0.5 × 10⁶ to 0.5 × 10⁸ CFU/ml was combined with the culture solution of the *Lactobacillus fermentum* strain (KCCM11910P) prepared in Example 9 at a concentration of 0.5 × 10⁶ to 0.5 × 10⁸ CFU/ml. The combination of the two strain culture solutions was administered orally to a man aged 28 and a man aged 54 who suffered from alopecia twice (50-100 ml each time) daily over 1-3 months.

The numbers of hairs lost were counted before and 10, 20, and 40 days after taking the strains. The patients were instructed to wash their hair before hair counting.

The experimental results are shown in Table 9.

**[Table 9]**

| Numbers of hairs lost before and after taking | | |
|---|---|---|
| **Subject Time** | **Man at age 28** | **Man at age 54** |
| Before taking | 43 hairs lost | 51 hairs lost |
| 10 days after taking | 24 hairs lost | 41 hairs lost |
| 20 days after taking | 14 hairs lost | 30 hairs lost |
| 40 days after taking | 9 hairs lost | 14 hairs lost |

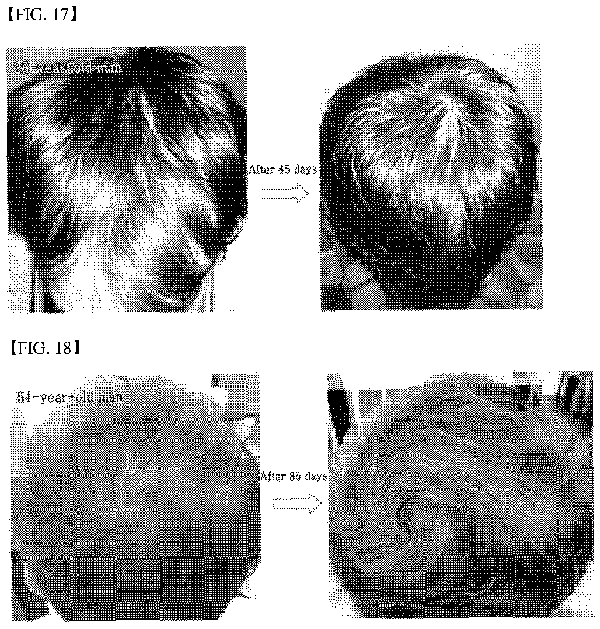

The hairs of the patients before and at the predetermined time points after administration of the strains were photographed to measure their thicknesses, numbers, and glosses. Some of the results are shown in FIGs. 17-18.

## Claims

1. A composition for preventing hair loss or promoting hair growth comprising a strain having lipolysis ability.

2. The composition according to claim 1, wherein the strain having lipolysis ability is a lactic acid bacterial strain isolated from kimchi.

3. The composition according to claim 2, wherein the lactic acid bacterial strain isolated from kimchi is *Leuconostoc holzapfelii, Leuconostoc mesenteroides* or *Lactobacillus sakei.*

4. The composition according to claim 3, wherein the lactic acid bacterial strain isolated from kimchi is *Leuconostoc holzapfelii* Ceb-kc-003 (KCCM11830P), *Leuconostoc mesenteroides* (KCCM11827P) or *Lactobacillus sakei* (KCCM11841P).

5. The composition according to claim 1, wherein the strain having lipolysis ability is administered in an amount of about 0.0001 mg/kg to about 10 g/kg daily.

6. The composition according to claim 1, wherein the composition is a pharmaceutical or food composition.

7. The composition according to any one of claims 1 to 6, wherein the composition is administered orally or parenterally.

8. The composition according to any one of claims 1 to 6, wherein the strain having lipolysis ability at a concentration of 5 × 10⁴ to 5 × 10⁸ CFU/ml is administered 1 to 4 times (30 ml to 100 ml each time) daily.

9. The composition according to any one of claims 1 to 6, wherein the composition is effective in improving sexual function.

10. The composition according to any one of claims 1 to 6, further comprising a strain capable of lowering cholesterol levels.

11. The composition according to claim 10, wherein the strain capable of lowering cholesterol levels is derived from cheonggukjang.

12. The composition according to claim 11, wherein the strain derived from cheonggukjang is a strain belonging to the genus *Brevibacillus, Lactobacillus, Lactococcus, Propionibacterium, Enterocuccus* or *Bifidobacterium.*

13. The composition according to claim 12, wherein the strain derived from cheonggukjang is *Brevibacillus reuszeri* Ceb-ch-003 (KCCM11911P), *Enterocuccus faecium* Ceb-ch-001 (KCCM11909P) or *Lactobacillus fermentum* Ceb-ch-002 (KCCM11910P).

14. The composition according to claim 10, further comprising a pharmaceutically or sitologically acceptable carrier.

15. The composition according to any one of claims 1 to 6, wherein the strain having lipolysis ability is present in an amount of 0.05 to 80 wt%, based on the weight of the composition.

16. The composition according to claim 10, wherein the composition comprises 0.05 wt% to 50 wt% of the strain capable of lowering cholesterol levels.

17. The composition according to claim 10, wherein the composition is effective in lowering cholesterol levels and/or improving sexual function.

18. A *Leuconostoc mesenteroides* strain (KCCM11827P), a culture thereof, a concentrate thereof or a dried product thereof.

19. A *Lactobacillus sakei* strain (KCCM11841P), a culture thereof, a concentrate thereof or a dried product thereof.

20. The strain or the culture, concentrate or dried product thereof according to claim 18 or 19, wherein the strain is effective in preventing hair loss, promoting hair growth, improving sexual function or decomposing fat.

21. A method for preparing a composition for preventing hair loss or promoting hair growth, comprising preparing a strain having lipolysis ability, a culture thereof, a concentrate thereof or a dried product thereof and applying a pharmaceutically or sitologically acceptable carrier to the strain or the culture, concentrate or dried product thereof.
